# EUROPÄISCHE PATENTSCHRIFT

(11) **EP 1 169 023 B1**
(45) Veröffentlichungstag und Bekanntmachung des Hinweises auf die Patenterteilung: **21.01.2004**
(21) Anmeldenummer: 00914148.2
(22) Anmeldetag: 17.03.2000
(51) Int. Cl.: A61K 9/20

(54) **Verfahren zu Herstellung von PULVERFÖRMIGEn SOLUBILISATIONSHILFSSTOFFEn FÜR FESTE PHARMAZEUTISCHE DARREICHUNGSFORMEN**
Process for the prepartation of SOLUBILIZING AIDS IN POWDER FORM FOR SOLID PHARMACEUTICAL PRESENTATION FORMS
Procèdè de la preparation des AUXILIAIRES DE SOLUBILISATION PULVERULENTS POUR FORMES D'ADMINISTRATION PHARMACEUTIQUES SOLIDES

(30) Priorität: 25.03.1999 DE 19913606
(43) Veröffentlichungstag der Anmeldung: 09.01.2002
(73) Patentinhaber: BASF AKTIENGESELLSCHAFT, 67056 Ludwigshafen (DE)
(72) Erfinder: BERNDL, Gunther, D-67273 Herxheim (DE); BREITENBACH, Jörg, D-68199 Mannheim (DE); RUCHATZ, Folker, D-67433 Neustadt (DE); SANNER, Axel, D-67227 Frankenthal (DE); SACK, Heinrich, D-67454 Hassloch (DE)
(86) Internationale Anmeldenummer: PCT/EP2000/002382
(87) Internationale Veröffentlichungsnummer: WO 2000/057855

(56) Entgegenhaltungen:
- EP-A- 0 729 748
- WO-A-93/11749
- DE-A- 3 530 780

## Beschreibung

Die vorliegende Erfindung betrifft ein Verfahren zur Herstellung von pulverförmige Hilfsstoffen mit hoher Beladungsdichte an solubilisierend wirkenden oberflächenaktiven Substanzen zur Verwendung in festen pharmazeutischen Darreichungsformen, enthaltend ein pharmazeutisch akzeptables Polymer und eine flüssige oder halbfeste solubilisierend wirkende oberflächenaktive Substanz.

Die Auflösegeschwindigkeit vieler schwer wasserlöslicher Wirkstoffe kann durch Vermischung mit Polymeren wie zum Beispiel Polyvinylpyrrolidon erhöht werden. Die Vermischung kann beispielsweise durch Verreibung, Schmelzextrusion von Polymer-Wirkstoff-Gemischen, Copräzipitation, Sprühtrocknung von Polymer-Wirkstoff-Lösungen oder Granulierung von Wirkstoff-Polymer-Gemischen im Wirbelbett oder durch Feuchtextrusion erfolgen. Häufig jedoch ist die Auflösegeschwindigkeit und die Bioverfügbarkeit solcher Polymer-Wirkstoff-Mischungen nicht ausreichend.

Es ist allgemein bekannt, daß man die Auflösegeschwindigkeit und die Bioverfügbarkeit durch Zugabe einer oberflächenaktiven Substanz erhöhen kann.

Aus der US-A 5,834,472 ist beispielsweise bekannt, daß durch Mitverwendung einer nichtionischen oberflächenaktiven Substanz die Bioverfügbarkeit eines speziellen Antifungicids erhöht werden kann.

In der WO 93/11749 ist ein Verfahren zur Herstellung fester Dispersionen schwer wasserlöslicher Wirkstoffe beschrieben, bei dem zunächst Wirkstoff und polymerer Träger vermischt werden und diese Mischung anschließend mit einer Lösung einer oberflächenaktiven Substanz im Wirbelbett granuliert wird. Die entstehenden Granulate werden dann mit Hilfe eines Extruders mit Heizzone extrudiert, gemahlen und zu Arzneiformen verarbeitet.

Aus der DE-A 35 30 780 sind Zubereitungen eines pharmazeutischen Wirkstoffs bekannt, welche durch Mischen des Wirkstoffs mit einem spezifischen Dispergiermittel sowie beispielsweise Polyethylenglykol in einem nicht-wässrigen Lösungsmittel, gegebenenfalls in Gegenwart eines inerten Trägermaterials, erhalten wird.

Aus der EP-A 729 748 sind pharmazeutische Zubereitungen bekannt, die durch Mischen des pharmazeutischen Wirkstoffs mit weiteren Additiven wie inerten Trägerstoffen und Dispergierhilfsmitteln in einem Extruder erhalten werden.

In der WO 93/11749 ist die Herstellung von pharmazeutischen Zubereitungen unter Verwendung von PVP als polymerem Trägermaterial und Solubilisatoren wie beispielsweise Polyethylenglykolen durch Schmelzextrusion beschrieben.

Eine Vielzahl von oberflächenaktiven Substanzen mit solubilisierenden Eigenschaften sind jedoch flüssig oder halbfest. Derartige Lösungsvermittler werden im allgemeinen in Formulierungen eingesetzt, die zur Abfüllung in Hart- oder Weichgelatinekapseln bestimmt sind oder in Lösungen zur intravenösen oder oralen Applikation.

Die Verwendung solcher Lösungsvermittler in für die Solubilisation schwerlöslicher Wirkstoffe relevanten Mengen von größer 10 Gew.-%, bezogen auf das Tablettengewicht, bereitet aufgrund der wachsartigen Konsistenz jedoch Probleme hinsichtlich der Verarbeitbarkeit der Formulierungen.

Aufgabe der vorliegenden Erfindung war es, eine Vorgehensweise zu finden, die den Einsatz größerer Mengen an flüssigen oder halbfesten solubilisierend wirkenden oberflächenaktiven Substanzen erlaubt, ohne daß die verarbeitungstechnischen Nachteile auftreten.

Die Aufgabe wurde durch das in Anspruch genannte Verfahren gelöst.

Das Verfahren dient zur Herstellung von pulverförmigen Hilfsstoffen zur Verwendung in festen pharmazeutischen Darreichungsformen, wobei die Hilfsstoffe als pharmazeutisch akzeptables polymeres Trägermaterial ein Homo- oder Copolymer des N-Vinylpyrrolidons und als Lösungsvermittler für die Solubilisation schwerlöslicher Wirkstoffe ein Umsetzungsprodukt von Ethylenoxid mit Ricinusöl, hydriertem Rizinusöl oder 12-Hydroxystearinsäure enthalten, welches bei 20°C flüssig ist oder einen Tropfpunkt von 20 bis 60 °C aufweist, dadurch gekennzeichnet, dass, bezogen auf die Gesamtmenge des Hilfsstoffs, mehr als 10 und bis zu 50 Gew.-% des Lösungsvermittlers eingesetzt werden und die Hilfsstoffe als freifließende Pulver durch Sprühtrocknung einer Lösung des polymeren Trägermaterials und des Lösungsvermittlers in Wasser, Ethanol, Isopropanol, Butanol, Aceton oder Mischungen davon als Lösungsmittel, erhalten werden, oder durch Verarbeitung der Inhaltsstoffe in einem Extruder zu einer homogenen Schmelze, die zu Pulvern verarbeitet wird.

Demgemäß wurde ein pulverförmiger Hilfsstoff, enthaltend neben einem pharmazeutisch akzeptablen Polymer mehr als 10 und bis zu 50 Gew.-%, bevorzugt 15 bis 40 Gew.-%, besonders bevorzugt 20 bis 30 Gew.-%, bezogen auf die Gesamtmenge des Hilfsstoffs, einer flüssigen oder halbfesten solubilisierend wirkenden oberflächenaktiven Substanz als Lösungsvermittler.

Flüssig oder halbfest bedeutet im Sinne dieser Erfindung, daß die oberflächenaktive Substanz bei 20°C flüssig ist bzw. einen Tropfpunkt im Bereich von 20 bis 60°C, bevorzugt 20 bis 50°C, besonders bevorzugt 20 bis 40°C aufweist. Die oberflächenaktive Substanz weist bevorzugt einen HLB-Wert (Hydrophilic Lipophilic Balance) im Bereich von 2 bis 18, besonders bevorzugt von 10 bis 15 auf.

Oberflächenaktive Substanz sind folgende nichtionischen Verbindungsklassen :

Solubilisatoren sind Umsetzungsprodukte von wechselnden Mengen Ethylenoxid mit Ricinusöl, hydriertem Ricinusöl oder 12-Hydroxystearinsäure, beispielsweise Polyoxyethylenglycerolricinolat-35, Polyoxyethylenglyceroltrihydroxystearat oder PEG-660-12-Hydroxystearinsäure(Polyglykolester der 12-Hydroxystearinsäure mit 30 mol-% Ethylenglykol.

Ebenso eignen sich Macrogol-6-cetylstearylether oder Macrogol-25-cetylstearylether.

Pharmazeutisch akzeptables polymeres Trägermaterial für den erfindungsgemäßen Hilfsstoff sind Homo- oder Copolymere des N-Vinylpyrrolidons mit K-Werten nach Fikentscher von 12 bis 100, bevorzugt 17 bis 30, beispielsweise Polyvinylpyrrolidon, Copolymere mit Vinylacetat oder Vinylpropionat wie beispielsweise Copovidon (VP/VAc-60/40).

Die Herstellung der Hilfsstoffe kann auf verschiedene Weisen erfolgen. So kann man beispielsweise den Solubilisator zu einer Lösung des Polymers geben und das Lösungsmittel anschließend entfernen. Als Lösungsmittel kommt insbesondere Wasser, aber auch Ethanol oder längerkettige Alkohole wie Isopropanol, Propanol, Butanole oder auch Aceton oder Mischungen solcher Lösungsmittel in Betracht. Bevorzugtes Trocknungsverfahren ist die Sprühtrocknung.

Weiterhin können die Hilfsstoffe nach an sich bekannten Granulierverfahren wie beispielsweise der Wirbelbettgranulation, wobei eine den Solubilisator enthaltende flüssige Phase auf den festen Träger aufgesprüht wird.

Die pulverförmigen Hilfsstoffe lassen sich weiterhin auch durch Schmelzextrusion in Abwesenheit von Lösungsmitteln herstellen. Bei der Schmelzextrusion kann die flüssige Solubilisatorphase kontinuierlich oder diskontinuierlich in den Extruder eindosiert werden. Die so erhaltenen Schmelze kann auf verschiedene Weise zu Pulvern verarbeitet werden.
So kann das strangförmig durch eine Düse oder Lochplatte austretende Extrudat durch übliche Abschlagtechniken, insbesondere dem Heissabschlag granuliert und gegebenenfalls noch vermahlen werden. Man kann die Schmelze auch über den offenen Extruderkopf ausfahren, wobei ebenfalls Granulate entstehen. Der Solubilisator enthaltende Hilfsstoff kann auch durch Kalandrierung zu Tabletten gepreßt und dann vermahlen werden. Zusätzlich kann die Vermahlung im Extruder erfolgen oder eine Granulierung über sogenannte Walzenstühle.

Gewünschtenfalls können die erfindungsgemäßen solubilisatorhaltigen Pulver auch noch weitere Hilfsstoffe enthalten, beispielsweise Fließregulierungsmitteln, Farbstoffe, Formentrennmittel, Fette und Wachse, Sprengmittel, Füllstoffe und weitere übliche Tablettierhilfsstoffe, wie beispielsweise Zucker, Zuckeralkohole oder Stärkeabbauprodukte.

Die erfindungsgemäßen Pulver sind freifließend und weisen vorzugsweise Korngrößen von 10 bis 1000 µ auf.

Sie können uneingeschränkt zur Herstellung von festen oral applizierbaren Formen wie Tabletten, Mikrotabletten, Sachetfüllungen, Brausetabletten, Lutschtabletten, Pellets oder Pastillen verarbeitet werden. Solche Formen können nach den gängigen pharmazeutischen Verfahren wie Schmelzextrusion, Tablettierung durch Verpressen oder Pastenextrusion mit anschließender Formgebung.

Die erfindungsgemäßen Pulver eignen sich grundsätzlich für Formulierungen sämtlicher pharmazeutischer, kosmetischer oder dietätischer Wirkstoffe. Insbesondere eignet er sich für Fomulierungen von ZNS-aktiven Substanzen, Dihydropyridinderivaten, Protease-Inhibitoren, Reverse-Transskriptase-Inhibitoren, Antimykotika oder Zytostatika. Besonders vorteilhaft an den erfindungsgemäßen Pulvern ist auch, daß weitere flüssige Substanzen wie z.B. Öle in den pulverförmigen Hilfsstoff eingearbeitet werden können und dann insbesondere bei öllöslichen Wirkstoffen zu einer Verbesserung der Bioverfügbarkeit führen.

### Beispiele

### Beispiel 1

In 5 l einer 20 %igen wäßrigen Lösung (m/V) von Polyvinylpyrrolidon mit einem K-Wert von 30 (Kollidon 30) wurden 1,65 l einer 20 %igen wäßrigen Lösung (m/V) von Cremophor RH 40 (Umsetzungsprodukt von 1 mol hydriertem Ricinusöl mit 45 mol Ethylenoxid) bei Raumtemperatur eingerührt. Die dabei erhaltene Lösung wurde anschließend sprühgetrocket. Man erhielt ein feines Pulver.

### Beispiel 3

In einen Zweischneckenextruder (ZSK 30 Werner & Pfleiderer) wurde mit 2 kg/h eines Copolymers aus 60 Gew.-% Vinylpyrrolidon und 40 Gew.-% Vinylacetat mit einem K-Wert von 30 mittels einer Waage zudosiert. Gleichzeitig wurde über eine Pumpe geschmolzenes Cremophor RH 40 kontinuierlich am Schuß 3 in den Extruder mit einer Pumprate von 0,5 kg/h zudosiert. Die Mischung wurde im Extruder homogenisiert und plasifiziert und anschließend mittels Kalander kalandriert.
Temperaturen [°C-9: 30 78 120 109 110 110
Düse [°C] : 103
Vakuum: 80 mbar
Die kalandrierten Formlinge wurden mittels einer Ringsiebmühle der Fa. Retsch (Sieb 2 mm) vermahlen.

### Tablettierung

50 Gew.-% des erhaltenen Pulvers wurde mit 10 Gew.-% Crospovidone, 10 Gew.-% Ca-Silikat, 8,5 Gew.-% microkristalline Cellulose, 20 Gew.-% Ciclosporin, 0,5 Gew.-% Mg-Stearat und 1 Gew.-% Aerosil (hochdisperse Kieselsäure) zu 500 mg Tabletten verpreßt.

### Beispiel 3

In einen Zweischneckenextruder (ZSK 30 Werner & Pfleiderer) wurde mit 2 kg/h ein Copolymer aus 60 Gew.-% Vinylpyrrolidon und 40 Gew.-% Vinylacetat mit einem K-Wert von 30 mittels einer Waage zudosiert. Gleichzeitig wurde über eine Pumpe geschmolzenes Cremophor RH 40 im Gemisch mit 20 Gew.-% Maisöl kontinuierlich am Schuß 3 in den Extruder mit einer Pumprate von 0,5 kg/h zudosiert. Die Mischung wurde im Extruder homogenisiert und plastifiziert und anschließend mittels Kalander kalandriert.
Die konfektionierte Mischung enthielt:
80 Gew.-% Kollidon VA 64 (Copovidon)
16 Gew.-% Cremophor RH 40
4 Gew.-% Maisöl
Temperaturen [o]: 30 70 115 105 105 105
Düse (°C] : 103
Vakuum: 80 mbar

## Patentansprüche

1. Verfahren zur Herstellung von pulverförmigen Hilfsstoffen zur Verwendung in festen pharmazeutischen Darreichungsformen, wobei die Hilfsstoffe als pharmazeutisch akzeptables polymeres Trägermaterial ein Homo- oder Copolymer des N-Vinylpyrrolidons und als Lösungsvermittler für die Solubilisation schwerlöslicher Wirkstoffe ein Umsetzungsprodukt von Ethylenoxid mit Ricinusöl, hydriertem Rizinusöl oder 12-Hydroxystearinsäure enthalten, welches bei 20°C flüssig ist oder einen Tropfpunkt von 20 bis 60 °C aufweist, **dadurch gekennzeichnet, dass**, bezogen auf die Gesamtmenge des Hilfsstoffs, mehr als 10 und bis zu 50 Gew.-% des Lösungsvermittlers eingesetzt werden und die Hilfsstoffe als freifließende Pulver durch Sprühtrocknung einer Lösung des polymeren Trägermaterials und des Lösungsvermittlers in Wasser, Ethanol, Isopropanol, Butanol, Aceton oder Mischungen davon als Lösungsmittel, erhalten werden, oder durch Verarbeitung der Inhaltsstoffe in einem Extruder zu einer homogenen Schmelze, die zu Pulvern verarbeitet wird.

2. Verfahren nach Anspruch 1, **dadurch gekennzeichnet, dass** der Lösungsvermittler einen Tropfpunkt von 20 bis 40 °C aufweist.

3. Verfahren nach Anspruch 1 oder 2, **dadurch gekennzeichnet, dass** der Lösungsvermittler einen HLB-Wert von 10 bis 15 aufweist.

## Claims

1. A process for producing excipients in powder form for use in solid pharmaceutical presentations, where the excipients comprise as pharmaceutically acceptable polymeric carrier material a homo- or copolymer of N-vinylpyrrolidone and as solubilizer for solubilizing slightly soluble active ingredients a product of the reaction of ethylene oxide with castor oil, hydrogenated castor oil or 12-hydroxystearic acid, which is liquid at 20°C or has a drop point of from 20 to 60°C, wherein more than 10 and up to 50% by weight of the solubilizer, based on the total amount of excipient, are employed, and the excipients are obtained as free-flowing powders by spray-drying a solution of the polymeric carrier material and of the solubilizer in water, ethanol, isopropanol, butanol, acetone or mixtures thereof as solvents, or by processing the constituents in an extruder to give a homogeneous melt which is processed to powders.

2. A process as claimed in claim 1, wherein the solubilizer has a drop point of from 20 to 40°C.

3. A process as claimed in claim 1 or 2, wherein the solubilizer has an HLB of from 10 to 15.

## Revendications

1. Procédé pour la préparation de produits auxiliaires pulvérulents à utiliser dans des formes d'administration pharmaceutiques solides, selon lequel les produits auxiliaires contiennent en tant que véhicule polymère acceptable pour l'usage pharmaceutique un homo- ou co-polymère de la N-vinylpyrrolidone et en tant que solvant servant à la solubilisation de substances actives peu solubles un produit de réaction de l'oxyde d'éthylène sur l'huile de ricin, l'huile de ricin hydrogénée ou l'acide 12-hydroxystéarique, produit de réaction qui est liquide à 20°C ou présente un point de goutte de 20 à 60°C, **caractérisé en ce que**, par rapport à la quantité totale du produit auxiliaire, on met en oeuvre l'agent solubilisant en proportions supérieures à 10 % et pouvant aller jusqu'à 50 % en poids et on obtient les produits auxiliaires à l'état de poudres qui s'écoulent librement par séchage par atomisation d'une solution du véhicule polymère et de l'agent solubilisant dans l'eau, l'éthanol, l'isopropanol, le butanol, l'acétone ou leurs mélanges servant de solvants, ou par conversion des constituants, dans une extrudeuse, en une masse fondue homogène qu'on met à l'état de poudre.

2. Procédé selon la revendication 1, **caractérisé en ce que** l'agent solubilisant a un point de goutte de 20 à 40°C.

3. Procédé selon la revendication 1 ou 2, **caractérisé en ce que** l'agent solubilisant a une valeur HLB de 10 à 15.
